# EUROPÄISCHE PATENTSCHRIFT

(11) **EP 0 639 363 B1**
(45) Veröffentlichungstag und Bekanntmachung des Hinweises auf die Patenterteilung: **01.04.1998**
(21) Anmeldenummer: 94111601.4
(22) Anmeldetag: 26.07.1994
(51) Int. Cl.: A61F 13/20, B30B 7/04

(54) **Tampon sowie Verfahren und Vorrichtung zu seiner Herstellung**
Tampon and a process and apparatus for producing it
Tampon, procédé et dispositif pour sa production

(30) Priorität: 28.07.1993 DE 4325220
(43) Veröffentlichungstag der Anmeldung: 22.02.1995
(73) Patentinhaber: KARL RUGGLI AG, CH-8435 Fisibach (CH)
(72) Erfinder: Brinker, Alfred Dr., D 58285 Gevelsberg (DE)
(74) Vertreter: Stenger, Watzke & Ring Patentanwälte

(56) Entgegenhaltungen:
- DE-A- 3 606 150
- DE-A- 3 934 153
- FR-A- 2 312 364
- US-A- 4 951 368

## Beschreibung

Die Erfindung betrifft einen insbesondere für die Frauenhygiene bestimmten Tampon aus einem durch Aufwickeln eines bandförmigen Faservliesabschnittes hergestellten zylindrischen Rohling, der durch Beaufschlagen von Preßkraft auf gleichmäßig über seinen Umfang verteilte Mantelabschnitte zu einem Vorformling mit einem Kern mit höherer Verdichtung des Fasermaterials und den Kern umgebenden Längsrippen mit niedrigerer Verdichtung umgeformt und durch Aufbringen radialer Druckkraft auf die Längsrippen zum fertigen Tampon endverformt ist. Weiterhin betrifft die Erfindung ein Verfahren und eine Vorrichtung zur Herstellung eines derartigen Tampons.

Aus der DE-PS 39 34 153 ist ein Tampon der voranstehend beschriebenen Art bekannt. Dieser Tampon hat bei gleicher Knickfestigkeit und Saugleistung gegenüber Tampons mit über den gesamten Tamponquerschnitt gleicher Verdichtung des Fasermaterials den Vorteil eines geringeren Materialeinsatzes. Die Saugleistung eines Tampons ergibt sich aus der von ihm bewirkten Sauggeschwindigkeit und seiner Flüssigkeitsaufnahmekapazität.

Aus der FR.A.23 12 364 bekannt ist ein Tampon, bei dem **im Endstadium** das Tamponmaterial im zentralen Bereich geringer verdichtet ist, als im Randbereich.

Zwar geht aus dieser Entgegenhaltung hervor, daß zu Beginn der Preßbewegung durch Verwendung eines ersten Satzes von ersten Preßbacken die Preßkräfte am zentralen Bereich des Tampons vorbeilaufen, und daß auch die Wirkungslinien der Preßkräfte eines zweiten Satzes von zweiten Preßbacken anfänglich am zentralen Bereich vorbeiweisen.

Aus dieser Engegenhaltung ergibt sich aber auch, daß während des letzten Bewegungsabschnittes der zweiten Preßbacken das Tamponmaterial im Zentrum des herzustellenden Tampons verdichtet wird.

Der Erfindung liegt die **Aufgabe** zugrunde, einen aus der DE-PS 39 34 153 bekannten Tampon sowie das Verfahren und die Vorrichtung zu seiner Herstellung derart weiterzubilden, daß bei Aufrechterhaltung der Knickfestigkeit eine Erhöhung der Saugleistung möglich wird.

Der diese Aufgabenstellung lösende Tampon nach der Erfindung ist dadurch gekennzeichnet, daß der Kern durch einen im Querschnitt ringförmigen Bereich höchster Verdichtung und einen innerhalb dieses ringförmigen Bereiches liegenden zentralen Bereich mit mittlerer Verdichtung gebildet ist.

Durch den Ersatz des insgesamt aus hochverdichtetem Faservliesmaterial bestehenden Kernes durch einen im Querschnitt ringförmigen Bereich höchster Verdichtung und einen innerhalb dieses ringförmigen Bereiches liegenden zentralen Bereich mittlerer Verdichtung bleibt einerseits die Formsteifigkeit des Tampons erhalten, da der ringförmige Querschnitt aus Fasermaterial höchster Verdichtung im wesentlichen dasselbe Flächenträgheitsmoment und damit in Verbindung mit dem Zentralbereich mittlerer Verdichtung dieselbe Steifigkeit hat wie ein Kernbereich gleichmäßiger Verdichtung. Andererseits wird aber durch die geringe Verdichtung des Fasermaterials im Zentralbereich des Tampons trotz geringeren Materialeinsatzes zumindest dieselbe Saugleistung erzielt, weil das Saugvolumen beibehalten und die Sauggeschwindigkeit im Zentralbereich durch dessen höhere Kapillarwirkung gesteigert wird.

Das erfindungsgemäße Verfahren erfolgt wie beim bekannten Stand der Technik durch Umformen des aus aufgewickeltem bandförmigen Faservliesmaterial bestehenden Rohlings zu einem Vorformling infolge Aufbringens von Preßkräften auf im Abstand zueinander liegende Mantelabschnitte und durch Endverformen des mit Längsrippen versehenen Vorformlings infolge Aufbringens radialer Druckkräfte. Die erfindungsgemäße Weiterbildung dieses Verfahrens ist dadurch gekennzeichnet, daß die Preßkräfte tangential auf einen die Längsmittelachse des Tampons mit vorgegebenem Abstand umgebenden Kreiszylinder gerichtet sind.

Durch die exzentrische Einleitung der Preßkräfte in den Rohling ergibt sich anstelle eines Kernbereiches gleicher Verdichtung beim erfindungsgemäßen Verfahren ein im Querschnitt ringförmiger Bereich höchster Verdichtung und ein Zentralbereich mittlerer Verdichtung. Die gegenüber dem bekannten Verfahren unterschiedliche Ausrichtung der Preßkräfte führte somit ohne größeren Aufwand zur Herstellung eines erfindungsgemäßen Tampons.

Die Vorrichtung zur Herstellung eines Tampons nach der Erfindung umfaßt in Übereinstimmung mit der bekannten Vorrichtung eine Anzahl von Preßwerkzeugen zur Verdichtung des Rohlings im Kernbereich und zur Erzeugung von Längsrippen im Mantelbereich sowie ein Formwerkzeug für die Endverformung des Tampons. Sie ist zur Lösung der der Erfindung zugrundeliegenden Aufgabenstellung dadurch gekennzeichnet, daß die Preßwerkzeuge jeweils am innenliegenden Ende eines Hebels angeordnet sind, der zwischen zwei Endstellungen um eine Schwenkachse verschwenkbar ist. Die Anordnung der Preßwerkzeuge am innenliegenden Ende von Hebeln schafft auf einfache Weise die Möglichkeit, die zur Schaffung des erfindungsgemäßen Tampons bzw. zur Durchführung des erfindungsgemäßen Verfahrens notwendigen Preßkräfte derart auszurichten, daß sie tangential auf einen die Längsmittelachse des Tampons mit vorgegebenem Abstand umgebenden Kreiszylinder gerichtet sind und auf diese Weise eine Aufteilung des Kernbereiches in einen im Querschnitt ringförmigen Bereich höchster Verdichtung und einen innerhalb dieses ringförmigen Bereiches liegenden Zentralbereich geringerer Verdichtung ergeben.

Bei einer bevorzugten Ausführungsform der erfindungsgemäßen Vorrichtung ist jeder Hebel an seinem außenliegenden Ende über einen Koppelhebel an einem Tragring gelagert und durch einen Verstellring verschwenkbar, an dem die Hebel jeweils mittels eines Lagerbolzens verschwenkbar gelagert sind. Hierdurch ergibt sich eine besonders einfache Konstruktion.

Gemäß weiteren Merkmalen der Erfindung wird vorgeschlagen, die Preßwerkzeuge auswechselbar an den Hebeln anzuordnen und mit hervorstehenden Preßschneiden zu versehen, um radial außerhalb des im Querschnitt ringförmigen Bereiches höchster Verdichtung längsverlaufende Rippen geringer Verdichtung und zwischen diesen Rippen verlaufende Längsnuten zu erzeugen, die nach der Endverformung des Tampons in der Art von Kammern im Außenbereich die Saugleistung des Tampons erhöhen.

Auf der Zeichnung sind ein Ausführungsbeispiel des Tampons und einer Vorrichtung zu seiner Herstellung dargestellt, und zwar zeigen:
- Fig. 1: eine Vorrichtung zur Herstellung eines Vorformlings als Bestandteil einer Maschine zur Herstellung eines Tampons,
- Fig. 2: den zentralen Teil der Vorrichtung in geöffneter Ausgangsstellung,
- Fig. 3: den zentralen Teil der Vorrichtung in einer Zwischenstellung,
- Fig. 4: den zentralen Teil der Vorrichtung in geschlossener Endstellung,
- Fig. 5: einen Querschnitt durch einen Tampon und
- Fig. 6: eine schematische Darstellung des Tamponquerschnittes nach Fig. 5.

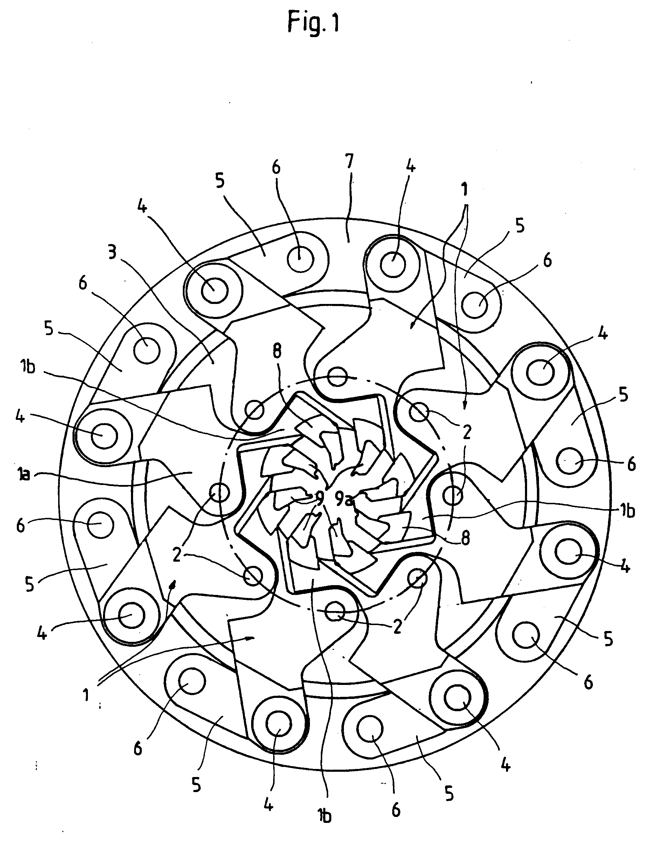

Die in Fig. 1 anhand eines Ausführungsbeispiels dargestellte Vorrichtung zur Herstellung eines Tampons umfaßt acht Hebel 1, die jeweils um einen Lagerbolzen 2 beschränkt verschwenkbar an einem Verstellring 3 gelagert sind. Jeder Hebel 1 ist an seinem radial außenliegenden Ende über einen Kupplungsbolzen 4 mit einem Ende eines Koppelhebels 5 gelenkig verbunden, dessen anderes Ende mittels eines Bolzens 6 an einem gestellfesten Tragring 7 verschwenkbar gelagert ist. Sowohl die Bolzen 6 als auch die Lagerbolzen 2 liegen jeweils auf einem Kreis, wobei der Abstand dieser Bolzen zueinander sich aus der durch die Anzahl der Hebel 1 vorgegebenen Teilung des jeweiligen Kreises ergibt.

Die in der Art eines Winkelhebels ausgebildeten Hebel 1, die zwischen ihrer Lagerung mittels des Lagerbolzens 2 auf dem Verstellring 3 und ihrer Anlenkung mittels des Kupplungsbolzens 4 am Koppelhebel 5 einen Kragarm 1a bilden, umfassen weiterhin einen radial einwärts weisenden Hebelarm 1b, der an seinem radial innenliegenden Ende eine Werkzeugaufnahme 8 trägt, an der ein Preßwerkzeug 9 befestigt ist. Jedes Preßwerkzeug 9 ist mit einer Preßschneide 9a ausgebildet, die im Zentrum der Fig. 1 zu erkennen sind.

Durch ein Verdrehen des konzentrisch zum gestellfesten Tragring 7 angeordneten Verstellringes 3 erfolgt ein Verschwenken der Hebel 1. Diese Hebel 1 werden gemäß Fig. 1 bei einem Verdrehen des Verstellringes 3 im Gegenuhrzeigersinn mit ihren Preßwerkzeugen 9 radial nach innen bewegt. Hierbei schwenken die Hebel 1 um die am Verstellring 3 angeordneten Lagerbolzen 2, wobei die über die Koppelhebel 5 mit dem Tragring 7 verbundenen Kupplungsbolzen 4 die Verschwenkbewegung bewirken, welche eine radial einwärts gerichtete Bewegung der Preßwerkzeuge 9 zur Folge hat. Auf diese Weise erfolgt ein "Schließen" der Preßwerkzeuge 9. Bei einer Verdrehung des Verstellringes 3 im Uhrzeigersinn erfolgt dementsprechend ein "Öffnen" der Preßwerkzeuge 9.

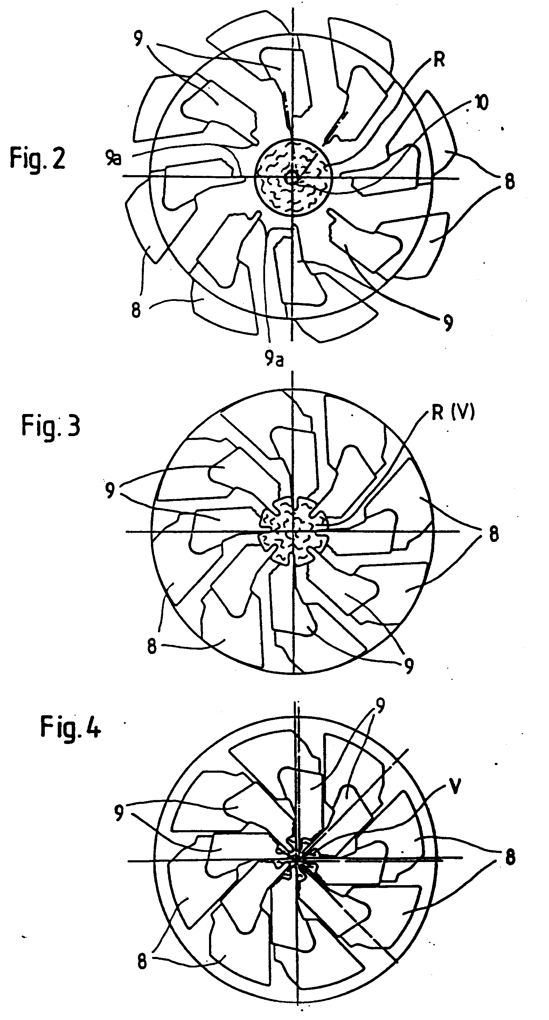

In Fig. 2 ist dargestellt, daß die Preßschneiden 9a der Preßwerkzeuge 9 in der geöffneten Ausgangsstellung nicht auf das Zentrum der Vorrichtung, sondern tangential auf einen die Längsmittelachse mit vorgegebenen Abstand umgebenden Kreiszylinder 10 gerichtet sind. Dieser Kreiszylinder 10 ist strichpunktiert in Fig. 2 eingezeichnet. Hierdurch wird erreicht, daß die von den Preßwerkzeugen 9 ausgeübten Preßkräfte nicht zentral, sondern tangential auf einen die Längsmittelachse des herzustellenden Tampons mit vorgegebenen Abstand umgebenden Kreis gerichtet sind. Diese exzentrische Ausrichtung der Preßschneiden 9a zum Mittelpunkt der Vorrichtung läßt sich durch die jeweilige Lage der Lagerbolzen 2, die Ausgestaltung der Hebel 1 sowie der Koppelhebel 5 beliebig einstellen.

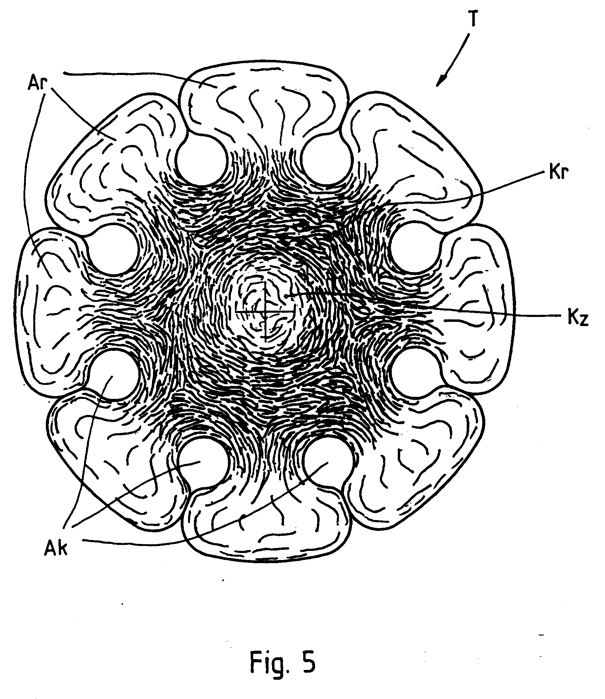

In der geöffneten Ausgangsstellung der Vorrichtung gemäß Fig. 2 wird ein zylindrischer Rohling R in den Raum zwischen die Preßwerkzeuge 9 eingesetzt. Dieser Rohling R ist durch Aufwickeln eines bandförmigen Faservliesabschnittes hergestellt worden. Durch Verdrehen des Verstellringes 3 relativ zum gestellfesten Tragring 7 im Gegenuhrzeigersinn werden die Preßwerkzeuge 9 zuerst in die in Fig. 3 dargestellte Zwischenstellung und schließlich in die in Fig. 4 dargestellte Endstellung überführt, in der sich die Vorrichtung in der geschlossenen Stellung befindet. Bei dieser Verschwenkbewegung der Hebel 1 werden diese einerseits mit dem Verstellring 3 bewegt und andererseits um die Lagerbolzen 2 des sich drehenden Verstellringes 3 durch die am feststehenden Tragring 7 angelenkten Koppelhebel 5 derart verschwenkt, daß die Preßwerkzeuge 9 eine kombinierte Bewegung mit tangentialer und radialer Komponente ausführen. Ein Vergleich der Fig. 3 und 4 miteinander und mit Fig. 2 ergibt, daß hierbei die durch die Preßwerkzeuge 9 und deren Preßschneiden 9a ausgeübten Verformungskräfte zur einer Verringerung des Volumens des Rohlings R führen, die über den Umfang gleichmäßig ist und den Rohling R zu einem Vorformling V (siehe Fig. 4) umformt, der einen Kern und diesen Kern umgebende Längsrippen aufweist. Ein derartiger Vorformling V ist vergrößert in Fig. 5 dargestellt. Nach geringfügigem Öffnen der Preßwerkzeuge 9 wird der Vorformling V der Vorrichtung entnommen und in nachgeschalteten Verfahrensschritten durch Aufbringen radialer Druckkräfte auf die Rippen zu einem Tampon endverformt.

Die Fig. 5 zeigt einen Querschnitt durch einen fertigen Tampon T. Dieser umfaßt einen Kernbereich unterschiedlicher Verdichtung, und zwar einen ringförmigen Bereich Kr höchster Verdichtung sowie einen zentralen Bereich Kz mittlerer Verdichtung. Dieser Kernbereich wird umgeben durch einen Außenbereich Ar, der durch die verformten Rippen des Vorformlings V entstanden ist, und zwar unter Einschluß von in Längsrichtung des Tampons verlaufenden Kammern Ak. In dem durch Verformen der Rippen des Vorformlings V entstandenen Außenbereich Ar ist die niedrigste Verdichtung des Faservliesmaterials anzutreffen. Es folgt im ringförmigen Bereich Kr die höchste Verdichtung; im zentralen Bereich Kz ist eine mittlere Verdichtung anzutreffen.

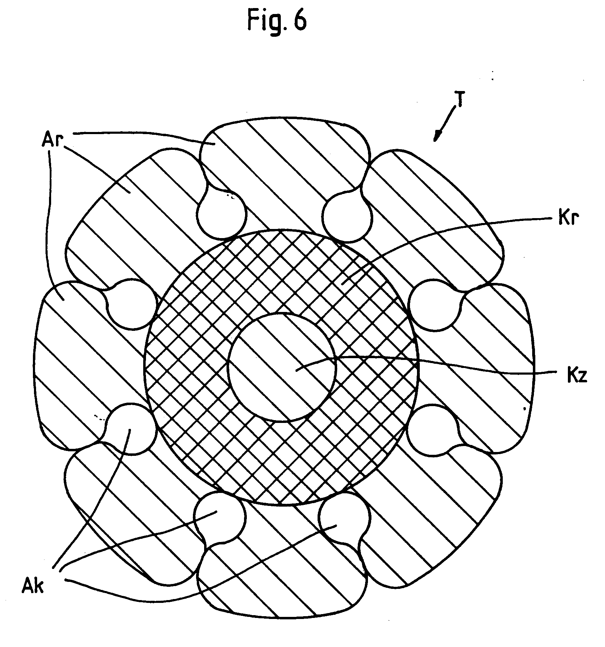

Diese unterschiedlichen Verdichtungen des fertigen Tampons T sind in Fig. 6 nochmals schematisch anhand eines Tamponquerschnitts dargestellt. Der ringförmige Bereich Kr weist die höchste Verdichtung auf, der innerhalb dieses ringförmigen Bereiches Kr liegende zentrale Bereiche Kz hat eine mittlere Verdichtung, wogegen das Faservliesmaterial im Außenbereich Kr die niedrigste Verdichtung hat. Die schematische Darstellung in Fig. 6 läßt erkennen, daß durch diesen Tamponaufbau, der durch die tangentiale Ausrichtung der Preßkräfte auf einen die Längsmittelachse des Tampons mit vorgegebenem Abstand umgebenden Kreiszylinder entsteht, folgende Vorteile erzielt werden: Durch den ringförmigen Bereich Kr höchster Verdichtung erhält der Tampon T zum einen eine hohe Formsteifigkeit, insbesondere Knicksteifigkeit. Der Tampon T umfaßt gleichermaßen ein integriertes Rohr, wie der 2-fach schraffierte Bereich in Fig. 6 erkennen läßt. Zum anderen wird der niedriger verdichtete zentrale Bereich Kz für die Aufnahme der Körperflüssigkeit nutzbar gemacht, die aufgrund der niedrigen Verdichtung im Außenbereich Ar und aufgrund des Vorhandenseins der längsverlaufenden Kammern Ak vom Tampon T sehr schnell aufgesaugt und durch den ringförmigen Bereich Kr bis in den zentralen Bereich Kz weitergeleitet wird, der aufgrund seiner mittleren Verdichtung eine gewisse Saugwirkung entfaltet.

### Bezugszeichenliste

- 1: Hebel
- 1a: Kragarm
- 1b: Hebelarm
- 2: Lagerbolzen
- 3: Verstellring
- 4: Kupplungsbolzen
- 5: Koppelhebel
- 6: Bolzen
- 7: Tragring
- 8: Werkzeugaufnahme
- 9: Preßwerkzeug
- 9a: Preßschneide
- 10: Kreiszylinder
- Ak: Kammer
- Ar: Außenbereich
- Kr: ringförmiger Bereich
- Kz: zentraler Bereich
- R: Rohling
- T: Tampon
- V: Vorformling

## Patentansprüche

1. Tampon, insbesondere für die Frauenhygiene, aus einem durch Aufwickeln eines bandförmigen Faservliesabschnittes hergestellten zylindrischen Rohling, der durch Beaufschlagen von Preßkraft auf gleichmäßig über seinen Umfang verteilte Mantelabschnitte zu einem Vorformling mit einem Kern mit höherer Verdichtung des Fasermaterials und den Kern umgebenden Längsrippen mit niedrigerer Verdichtung umgeformt und durch Aufbringen radialer Druckkräfte auf die Längsrippen zum fertigen Tampon endverformt ist,
**dadurch gekennzeichnet,**
daß der Kern durch einen im Querschnitt ringförmigen Bereich (Kr) höchster Verdichtung und einen innerhalb dieses ringförmigen Bereiches (Kr) liegenden zentralen Bereich (Kz) mit mittlerer Verdichtung gebildet ist.

2. Verfahren zur Herstellung eines Tampons nach Anspruch 1 durch Umformen des aus aufgewickeltem bandförmigen Faservliesmaterial bestehenden Rohlings zu einem Vorformling infolge Aufbringens von Preßkräften auf im Abstand zueinander liegende Mantelabschnitte und durch Endverformen des mit Längsrippen versehenen Vorformlings infolge Aufbringens radialer Druckkräfte,
**dadurch gekennzeichnet,**
daß die Preßkräfte tangential auf einen die Längsmittelachse des Tampons mit vorgegebenen Abstand umgebenden Kreiszylinder (10) gerichtet sind.

3. Vorrichtung zur Herstellung eines Tampons nach Anspruch 1 mit einer Anzahl von Preßwerkzeugen (9) zur Verdichtung des Rohlings (R) im Kernbereich und zur Erzeugung von Längsrippen im Mantelbereich sowie mit einem Formwerkzeug zur Endverformung des Tampons,
**dadurch gekennzeichnet,**
daß die Preßwerkzeuge (9) jeweils am innenliegenden Ende eines Hebels (1) angeordnet sind, der zwischen zwei Endstellungen um eine Schwenkachse (Lagerbolzen 2) verschwenkbar ist.

4. Vorrichtung nach Anspruch 3, dadurch gekennzeichnet, daß jeder Hebel (1) an seinem außenliegenden Ende über einen Koppelhebel (5) an einem Tragring (7) gelagert und durch einen Verstellring (3) verschwenkbar ist, an dem die Hebel (1) jeweils mittels eines Lagerbolzens (2) verschwenkbar gelagert sind.

5. Vorrichtung nach Anspruch 3 oder 4, dadurch gekennzeichnet, daß jedes Preßwerkzeug (9) auswechselbar am Hebel (1) angeordnet ist.

6. Vorrichtung nach mindestens einem der Ansprüche 3 bis 5, dadurch gekennzeichnet, daß jedes Preßwerkzeug (9) mit einer hervorstehenden Preßschneide (9a) ausgebildet ist.

## Claims

1. Tampon, in particular for feminine hygiene, comprising a cylindrical blank which is produced by winding up a section of nonwoven fabric in strip form and, by applying pressing force to sections of its outer surface evenly distributed over its circumference, is transformed into a preform with a core of higher compaction of the fibre material and longitudinal ribs of lower compaction, surrounding the core, and is finally formed into the finished tampon by applying radial compressive forces to the longitudinal ribs, characterized in that the core is formed by a cross-sectionally annular region (Kr) of highest compaction and a central region (Kz), lying within this annular region (Kr), of moderate compaction.

2. Process for producing a tampon according to Claim 1 by forming the blank comprising wound-up nonwoven fabric in strip form into a preform by applying pressing forces to sections of its outer surface spaced apart from one another and by final forming of the preform provided with longitudinal ribs, by applying radial compressive forces, characterized in that the pressing forces are directed tangentially at a circular cylinder (10) surrounding the longitudinal centre axis of the tampon at a prescribed distance.

3. Apparatus for producing a tampon according to Claim 1, having a number of pressing tools (9) for compacting the blank (R) in the core region and for producing longitudinal ribs in the outer surface region and also having a forming tool for the final forming of the tampon, characterized in that the pressing tools (9) are arranged respectively at the inner end of a lever (1) which can be swivelled between two end positions about a swivel axis (bearing bolt 2).

4. Apparatus according to Claim 3, characterized in that each lever (1) is mounted at its outer end via a coupling lever (5) on a carrying ring (7) and can be swivelled by an adjusting ring (3), at which the levers (1) are respectively swivel-mounted by means of a bearing bolt (2).

5. Apparatus according to Claim 3 or 4, characterized in that each pressing tool (9) is arranged replaceably on the lever (1).

6. Apparatus according to at least one of Claims 3 to 5, characterized in that each pressing tool (9) is designed with a protruding pressing edge (9a).

## Revendications

1. Tampon, en particulier pour l'hygiène féminine, composé d'une forme de départ cylindrique fabriquée par enroulement d'un coupon de matière de fibre non tissée sous forme d'une bande, qui, sous l'influence d'une force de compression exercée sur des sections d'enveloppe réparties de façon uniforme sur sa périphérie, est déformée en une préforme comportant un noyau, dans lequel la matière de fibre est plus tassée, et des nervures longitudinales entourant le noyau, dont le tassement est moindre, et qui, par application de forces de compression radiales sur les nervures longitudinales est transformée en tampon final, caractérisé en ce que,
le noyau est constitué d'une zone de section transversale annulaire (Kr) de tassement supérieur et d'une zone centrale (Kz) disposée à l'intérieur de cette zone annulaire (Kr), de tassement moyen.

2. Procédé de fabrication d'un tampon selon la revendication 1, par déformation de la forme de départ se composant de matière de fibre non tissée en forme de bande enroulée, en une préforme, à la suite de l'application de forces de compression sur des sections d'enveloppe espacées entre elles, et par déformation finale de la préforme dotée de nervures longitudinales, à la suite de l'application de forces de compression radiales, caractérisé en ce que,
les forces de compression sont appliquées tangentiellement sur un cylindre circulaire (10) entourant l'axe central longitudinal du tampon selon un écartement prédéterminé.

3. Dispositif pour la production d'un tampon selon la revendication 1, présentant un certain nombre d'outils de compression (9), destinés à tasser la forme de départ (R) dans la zone du noyau, et à produire des nervures longitudinales dans la zone de l'enveloppe, ainsi qu'un outil de formage pour la déformation finale du tampon,
caractérisé en ce que les outils de compression (9) sont chacun disposés à l'extrémité interne d'un levier (1), qui peut basculer autour d'un axe de pivotement (boulon d'appui 2) entre deux positions finales.

4. Dispositif selon la revendication 3, caractérisé en ce que chaque levier (1) est logé, au niveau de son extrémité externe, dans un anneau porteur (7) par un levier jumelé (5) et peut pivoter par l'intermédiaire d'une bague de réglage (3), dans laquelle les leviers (1) sont chacun logés de façon pivotante au moyen d'un boulon d'appui (2).

5. Dispositif selon la revendication 3 ou 4, caractérisé en ce que chaque outil de compression (9) est disposé sur le levier (1) de façon à pouvoir être remplacé.

6. Dispositif selon au moins une des revendications 3 à 5, caractérisé en ce que chaque outil de compression (9) est doté d'une lame de compression (9a) faisant saillie.
